(19) Europäisches Patentamt
European Patent Office
Office européen des brevets

(11) **EP 4 389 120 A1**

(12) **EUROPEAN PATENT APPLICATION**

(43) Date of publication:
**26.06.2024   Bulletin 2024/26**

(21) Application number: **22461646.6**

(22) Date of filing: **20.12.2022**

(51) International Patent Classification (IPC):
*A61K 31/135* (2006.01)   *A61K 31/485* (2006.01)
*A61K 33/06* (2006.01)   *A61K 45/06* (2006.01)
*A61P 43/00* (2006.01)

(52) Cooperative Patent Classification (CPC):
(C-Sets available)
**A61K 31/485; A61K 31/135; A61K 33/06;
A61K 45/06; A61P 43/00**   (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Inflacod Sp. z o.o.
62-002 Zlotniki (PL)**

(72) Inventor: **Bujalska-Zadrozny, Magdalena
03-029 Warszawa (PL)**

(74) Representative: **Witek, Rafal
WTS Patent Attorneys
Witek, Sniezko & Partners
ul. Weigla 12
53-114 Wroclaw (PL)**

(54) **AN ANTI-INFLAMMATORY PHARMACEUTICAL COMPOSITION**

(57)   The disclosed antiinflammatory pharmaceutical composition for oral administration is characterised in that it contains an opioid and a pharmaceutically admissible magnesium (II) compound, possibly along with one or more pharmaceutically admissible ancillary substances.

**(Cont. next page)**

EP 4 389 120 A1

(52) Cooperative Patent Classification (CPC): (Cont.)

C-Sets
**A61K 31/135, A61K 2300/00;**
**A61K 31/485, A61K 2300/00**

**Description**

The presented invention relates to a pharmaceutical composition for use in treatment of inflammation.

Inflammation is an essential immune response of living organisms that enables survival during infection or injury and maintains tissue homeostasis under a variety of noxious conditions. It is a comprehensive array of physiological responses to various pathogens (e.g., bacteria, viruses), dust particles, and and other factors able to cause damage to tissues [Medzhitov, 2010]. Inflammation contributes to many disorders such as trauma, arthritis, neuropathy, fibromyalgia, endometriosis, diabetes, cancer and chronic pain [Stein, 2013].

One of main symptoms of inflammation is pain which is mediated by inflammatory mechanisms that negatively affect patients' quality of life [Simon et al., 2021]. The treatment of inflammatory pain has always been a major goal in the clinic this pain type is related to several pathological conditions of inflammatory origin and surgical procedures, which are associated with inflammatory mediators. Despite some safety concerns, till date, the use of non-steroidal anti-inflammatory drugs (NSAIDs) is efficacious, relatively safe, and well tolerated by patients [Ronchetti et al., 2017]. It should be however noted that among many mechanisms underlying inflammatory pain, it has also been demonstrated that in these conditions analgesia might be also mediated through opioid receptor-mediated signaling pathway [Liu et al., 2021].

Opioids can produce potent analgesia by activating opioid receptors located within the central nervous system. This drug class can also relieve pain by mechanisms outside the central nervous system, thus avoiding centrally mediated unwanted effects. Peripheral opioid receptors are expressed in peripheral sensory neurons of the dorsal root ganglia and can interact with exogenous or endogenous opioid ligands both in animals and in humans. Inflammation of peripheral tissue leads to upregulation of these opioid receptors and to local production of endogenous opioid peptides in immune cells. A large number of studies have demonstrated that peripherally active opioids possess anti-inflammatory properties as they can reduce the release of proinflammatory neuropeptides, cytokines, plasma extravasation, vasodilation, immune mediators, expression of adhesion molecules and tissue destruction [Stein, 2013].

Unfortunately, the undesirable effects of opioids limit their utility. These are, amongst others: development of tolerance, chronic constipation, dizziness, disturbances of central nervous system, such as consciousness disorders, cognitive impairment or respiratory depression (particularly in the case of an overdose).

Also magnesium possesses some anti-inflammatory properties, partly because it antagonizes calcium and inhibits L-type calcium channels [Libako et al., 2016]. Moreover, magnesium induces the release of anti-inflammatory cytokines by inhibiting the TLR-NF-$\kappa$B signaling pathway [Zhang et al., 2019].

The aim of this invention is to create a pharmaceutical composition of anti-inflammatory drugs containing an opioid, possessing both analgesic as well as improved anti-inflammatory properties. It is particularly desirable to limit the daily dose of opioid and, consequently, to limit the risk of undesirable effects of opioid use.

Unexpectedly, such stated goals have been achieved by the present invention.

The subject of the present invention is an pharmaceutical composition for use in treatment of inflammation, comprising an opioid and a pharmaceutically admissible salt of magnesium (II), possibly with one or more pharmaceutically admissible ancillary substances, wherein said salt of magnesium (II) increase the anti-inflammatory activity of said opioid.

Examples of ancillary substances necessary for the manufacturing of an orally or other administered drug form, e.g. granulates (possibly in dosing sachets), sticks, tablets or capsules, patches, ointment, creams include fillers (e.g. sugars such as lactose, glucose or saccharose, alcohol sugar derivatives, such as mannitol, sorbitol, or xylitol, starches such as wheat, corn or potato starch), lubricants such as talcum, magnesium stearate, calcium stearate, colloidal silica or stearic acid, or binders such as polyvinylpyrrolidone or cellulose derivatives (carboxymethylcellulose, hydroxypropylcellulose, hydroxypropylmethylcellulose, methylcellulose) or gelatin. The tablets may be, for ease of use, coated with an appropriate film-form agent, e.g. hydroxypropylmethylcellulose, hydroxypropylcellulose, polivinyl alcohol or ethylcellulose, which may optionally be supplemented with ancillary substances, i.e. a softener such as glycerol, propylene glycol, diethyl phthalene or glycerol triacetate; a filler such as saccharose, sorbitol, xylitol, glucose or lactose; and a dye such as titanium dioxide, iron oxides, etc.

Preferably, the composition for use according to the invention is dedicated for oral administration.

Preferably, the composition for use according to the invention is characterised in that the compound of magnesium (II) is organic or inorganic form of magnesium (II), possibly in form of water soluble compound. Preferably, it is salt selected from a group consisting of: magnesium chloride, magnesium lactate, magnesium hydroaspartate, magnesium citrate or hydrates thereof. In particular, the salt of magnesium (II) is magnesium citrate or hydrate thereof, possibly magnesium citrate x 9 $H_2O$. Possibly, inorganic compound of magnesium (II) is magnesium oxide.

However, according to the present invention, using any other pharmaceutically admissible compound of magnesium (II) is acceptable, regardless of its hydration level or crystalline form (polimorphic/amorphic). According to the present invention, it is also possible to use pharmaceutically admissible magnesium (II) coordination complexes.

Preferably, the composition for use according to the invention is characterised in that the opioid is selected from a group consisting of: dihydrocodeine, oxycodone or phenantrene derivatives of opium such as: morphine or codeine. According to invention, using an atypical opioid (tramadol) and opioids from other groups is also acceptable.

Preferably, the composition for use according to the invention is characterised in that the opioid is in the form of pharmaceutically admissible salt thereof.

Preferably, the composition for use according to the invention is characterised in that the opioid is codeine, their salt or hydrate thereof, possibly codeine phosphate hemihydrate.

Preferably, the composition for use according to the invention is characterised in that the opioid is used in dose of 15 mg/kg to 240 mg/kg. Preferably, one dosage unit contains 15mg or 30 mg of codeine.

Preferably, the composition for use according to the invention is characterised in that the magnesium (II) is used in dose of 30 mg/kg to 300 mg/kg. Preferably, one dosage unit contains 150mg or 300 mg of magnesium (II).

Preferably, the composition for use according to the invention is characterised in that the inflammation is resulting from any ailments with an inflammatory component, in particular such as neuropathic pain, inflammatory pain, visceral pain.

Preferably, the composition for use according to the invention is characterised in that the inflammation is manifested by oedema.

Preferably, the composition for use according to the invention is characterised in that the inflammation is manifested by the inflammatory pain, in particular visceral or neuropathic pain such as pain resulting from inflamaton observed during diabetic-neuropathy.

According to the present invention, it unexpectedly turned out that orally administered magnesium (II) compound enhances the anti-inflammatory effect of a concomitantly administered opioid, in particular codeine or their pharmaceutically admissible salts.

In a particular version of the present invention, a pharmaceutical composition for use in treatment, such as an oral treatment, of inflammation composed of an opioid with a magnesium (II) compound also allows simplification of the dosage schedule (a patient takes two substances in one dosage unit, such as tablet, sachet or stick, instead of two independent units). Delivery of the composition in a single tablet makes it possible to use this combination not only in inpatient care but at home as well.

According to an alternative version of the present invention, a pharmaceutical composition for use in treatment of inflammation is possible in two separate forms, one containing an opioid and second containing an magnesium (II) compound.

In order to better demonstrate the concept of the present invention, this description is supplemented with the following figures:

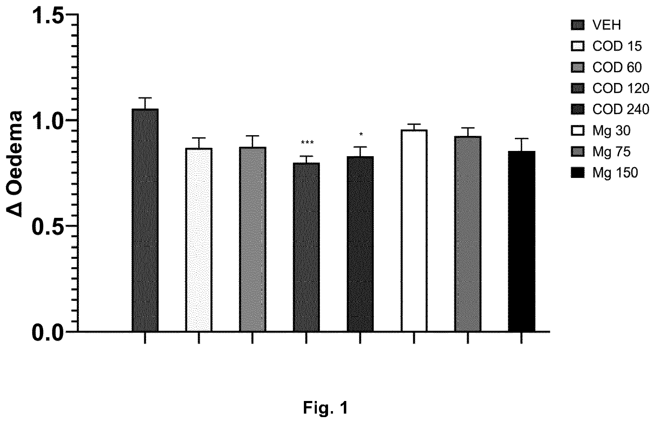

Figure 1 presents antioedematous activity of orally administered codeine (COD; 15, 60, 120 and 240 mg/kg) and magnesium (Mg; 30, 75 and 150 mg/kg) in the CARR-induced paw oedema model in rats (n=6-9). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH) at the respective time point of testing: * $p < 0.05$, *** $p < 0.001$. $\Delta$ Oedema - oedema increase at the respective time point of testing (3 h after CARR).

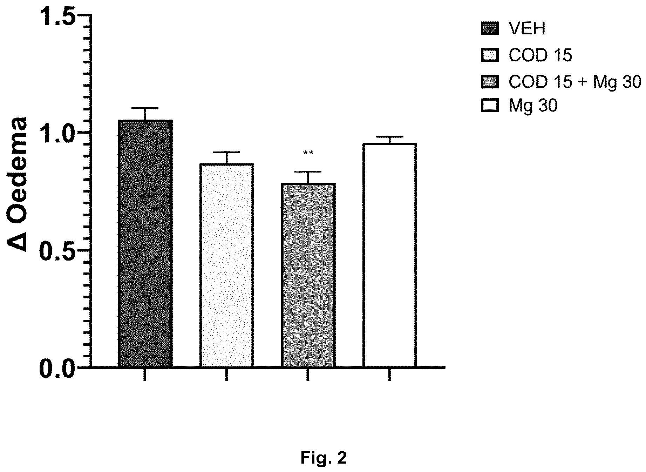

Figure 2 represents antioedematous activity of oral codeine (COD; 15 mg/kg) and magnesium (30 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): ** $p < 0.01$. $\Delta$ Oedema - oedema increase at the respective time point of testing (3 h after CARR).

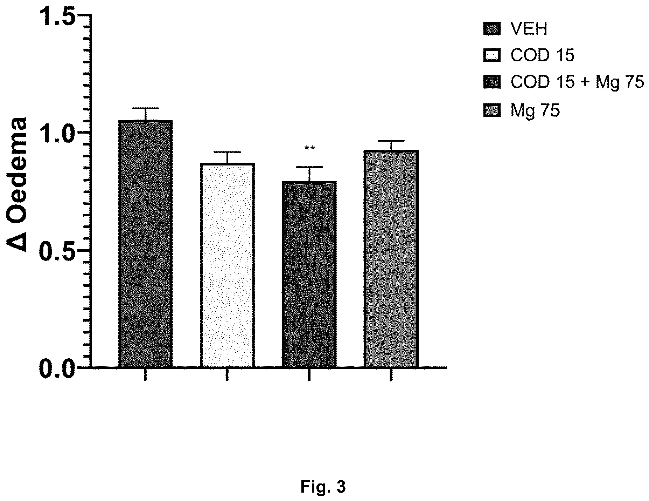

Figure 3 represents antioedematous activity of oral codeine (COD; 15 mg/kg) and magnesium (75 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): ** $p < 0.01$. $\Delta$ Oedema - oedema increase at the respective time point of testing (3 h after CARR).

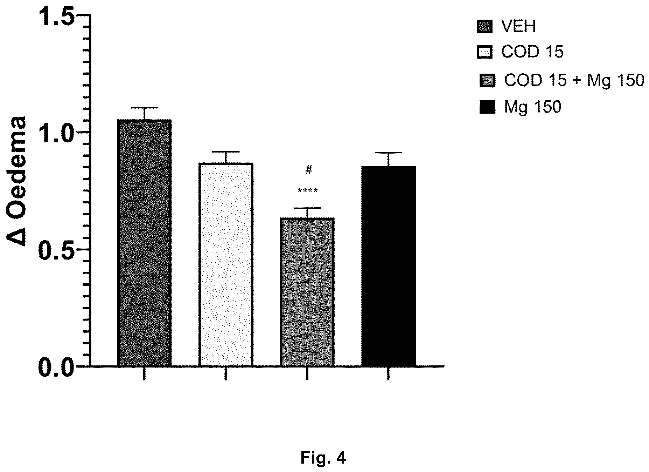

Figure 4 represents antioedematous activity of oral codeine (COD; 15 mg/kg) and magnesium (150 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): **** $p < 0.0001$; significance vs. COD 15 mg/kg used alone: # $p < 0.05$. $\Delta$ Oedema - oedema increase at the respective time point of testing (3 h after CARR).

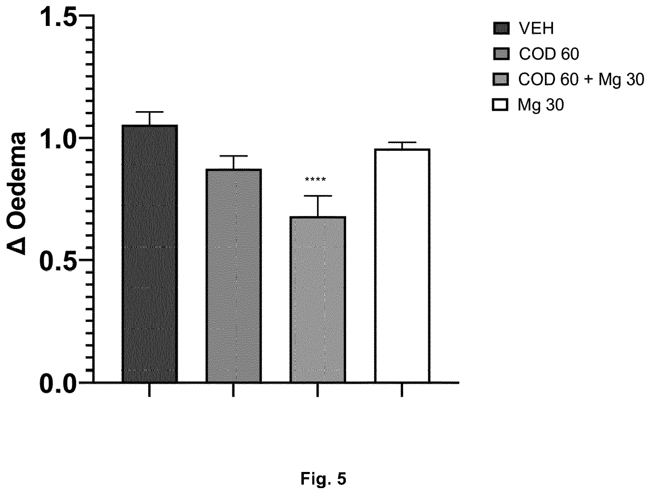

Figure 5 represents antioedematous activity of oral codeine (COD; 60 mg/kg) and magnesium (30 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): **** $p < 0.0001$. $\Delta$ Oedema - oedema increase at the respective time point of testing (3 h after CARR).

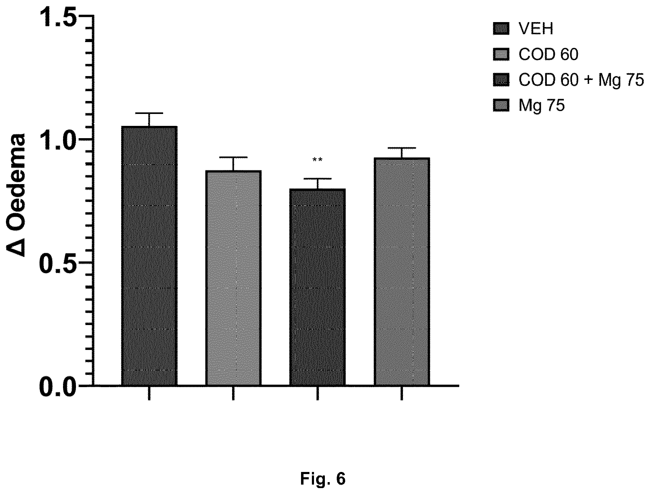

Figure 6 represents antioedematous activity of oral codeine (COD; 60 mg/kg) and magnesium (75 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): ** $p < 0.01$. $\Delta$ Oedema - oedema increase at the respective time point of testing (3 h after CARR).

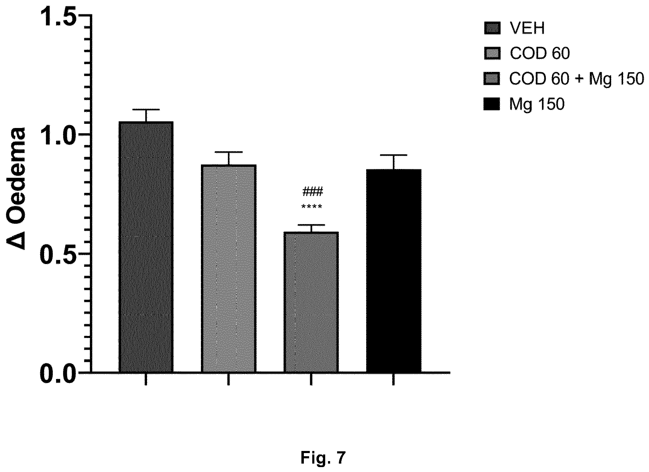

Figure 7 represents antioedematous activity of oral codeine (COD; 60 mg/kg) and magnesium (150 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): **** $p < 0.0001$; significance vs. COD 60 mg/kg used alone: ### $p < 0.001$. $\Delta$ Oedema - oedema increase at the respective time point of testing (3 h after CARR).

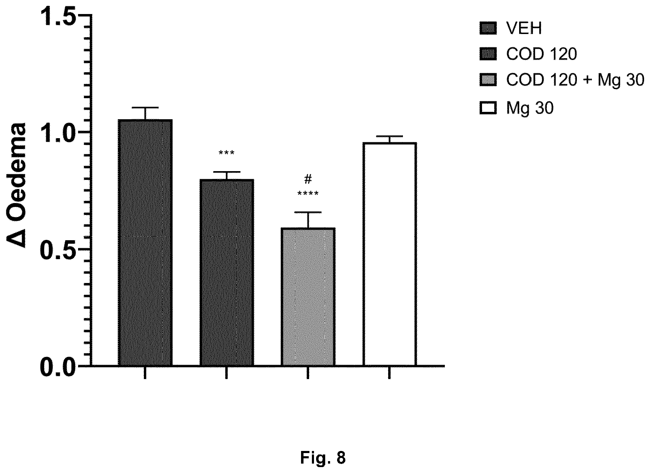

Figure 8 represents antioedematous activity of oral codeine (COD; 120 mg/kg) and magnesium (30 mg/kg) used alone or in combination in the CARR-induced paw oedema model in rats (n=6-9). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): *** $p < 0.001$, **** $p < 0.0001$; significance vs. COD 120 mg/kg used alone at the respective time point of testing: # $p < 0.05$. $\Delta$ Oedema - oedema increase at the respective time point of testing ((3 h after CARR)).

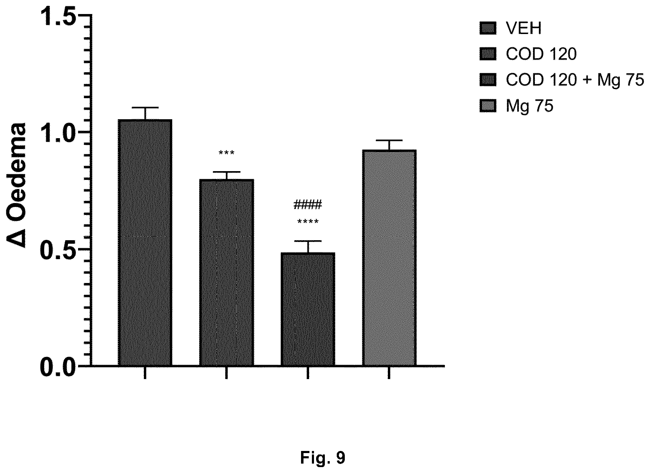

Figure 9 represents antioedematous activity of oral codeine (COD; 120 mg/kg) and magnesium (75 mg/kg) used alone or in combination in the CARR-induced paw oedema model in rats (n=6-9). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): *** $p < 0.001$, **** $p < 0.0001$; significance vs. COD 120 mg/kg used alone at the respective time point of testing: #### $p < 0.0001$. $\Delta$ Oedema - oedema increase at the respective time point of testing (3 h after CARR).

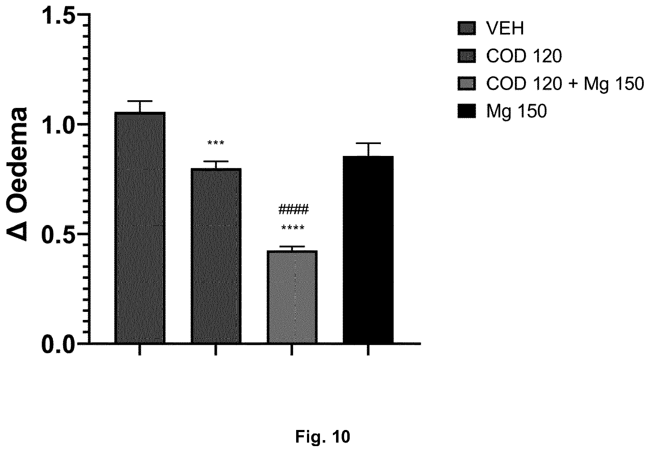

Figure 10 represents antioedematous activity of oral codeine (COD; 120 mg/kg) and magnesium (150 mg/kg) used alone or in combination in the CARR-induced paw oedema model in rats (n=6-9). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): *** $p < 0.001$, **** $p < 0.0001$; significance vs. COD 120 mg/kg used alone at the respective time point of testing: #### $p < 0.0001$. $\Delta$ Oedema - oedema increase at the respective time point of testing (3 h after CARR).

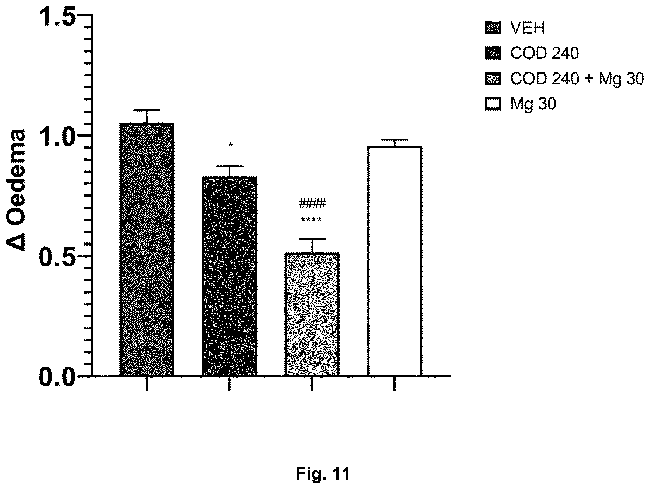

Figure 11 represents antioedematous activity of oral codeine (COD; 240 mg/kg) and magnesium (30 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): * $p < 0.05$, **** $p < 0.0001$; significance vs. COD 240 mg/kg used alone at the respective time point of testing: #### $p < 0.0001$. $\Delta$ Oedema - oedema increase at the respective time point of testing (3 h after CARR).

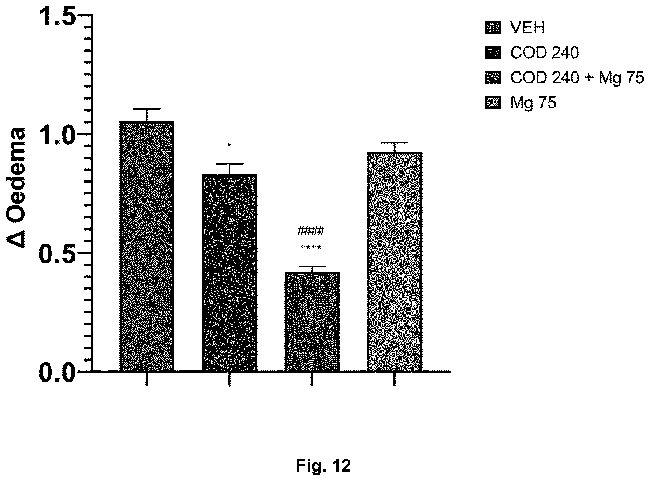

Figure 12 represents antioedematous activity of oral codeine (COD; 240 mg/kg) and magnesium (75 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): * $p < 0.05$, **** $p < 0.0001$; significance vs. COD 240 mg/kg used alone at the respective time point of testing: #### $p < 0.0001$. $\Delta$ Oedema - oedema increase at the respective time point of testing (3 h after CARR).

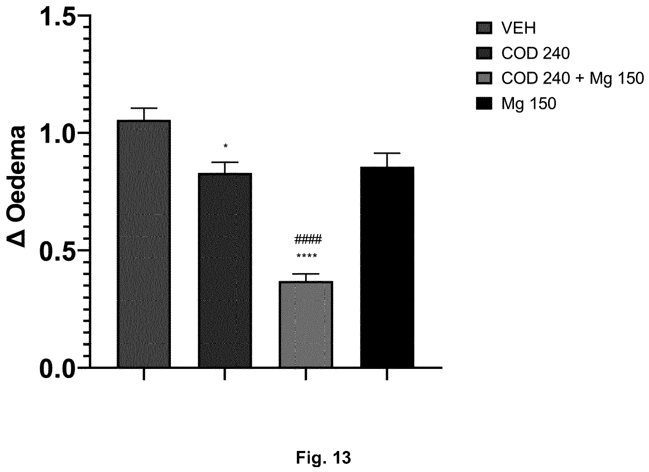

Figure 13 represents antioedematous activity of oral codeine (COD; 240 mg/kg) and magnesium (150 mg/kg) used alone or in combination in the CARR-induced paw oedema model in rats (n=6). Results are shown as the mean increase in paw volume compared to baseline value ($\pm$ SEM) and measured 3 h after intraplantar CARR injection. Statistical analysis: repeated measures ANOVA followed by Tukey's *post hoc* comparison. Significance vs. CARR+vehicle-treated group (VEH): * $p < 0.05$, **** $p < 0.0001$; significance vs. COD 240 mg/kg used alone at the respective time point of testing: #### $p < 0.0001$. $\Delta$ Oedema - oedema increase at the respective time point of testing (3 h after CARR).

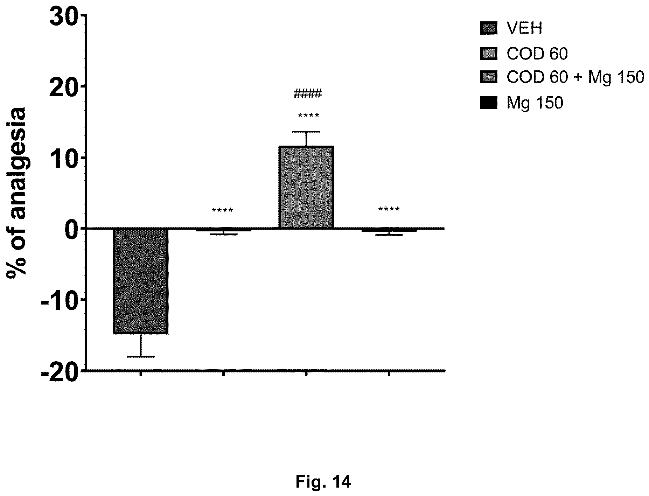

Figure 14 sumarises antinociceptive activity of orally administered codeine (COD; 60 mg/kg) and magnesium (Mg; 150 mg/kg), each used alone or in combination, in the CARR-induced paw oedema model in rats. Pain threshold was measured in the Randall-Selitto test 3 h after CARR administration. Results are shown as % of analgesia $\pm$ SEM (n = 6). Statistical analysis: repeated measures analysis of variance (ANOVA) followed by Tukey's multiple comparison. Significance vs. CARR+vehicle-treated group (VEH): **** $p < 0.0001$; vs. COD 60 mg/kg used alone: #### $p < 0.0001$.

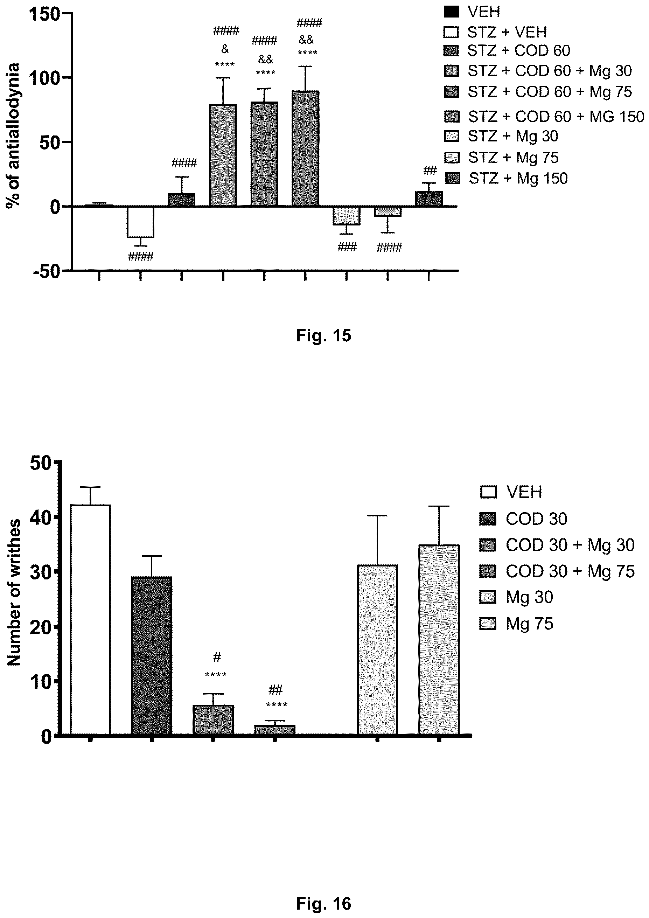

Figure 15 sumarises antiallodynic activity of orally administered codeine (COD; 60 mg/kg) combined with magnesium (Mg, dose 150 mg/kg) in the diabetic neuropathic pain model and measured using the von Frey test. Results are shown as % of antiallodynia ($\pm$ SEM) in response to mechanical stimulation. Statistical analysis: one-way analysis of variance (ANOVA), followed by Tukey's post hoc comparison. Significance vs. STZ+vehicle-treated group (VEH) group: **** $p < 0.0001$; significance vs. codeine 60 mg/kg used alone: & $p < 0.05$, && $p < 0.01$. Significance vs. normoglycemic control (VEH): ## $p < 0.01$, ### $p < 0.001$, #### $p < 0.0001$.

Figure 16 presents antinociceptive activity of orally administered codeine (COD; 30 mg/kg) and magnesium (Mg; 30, 75 mg/kg) used alone or in combination in the acetic acid-induced writhing test in mice. Results are shown as the number of writhing episodes (n = 8-10). Statistical significance was calculated using one-way ANOVA followed by Tukey's post hoc comparison. Significance vs. acetic acid+vehicle-treated group (VEH): **** $p < 0.0001$, and significance vs. COD 30 mg/kg used alone: # $p < 0.05$, ## $p < 0.01$.

Furthermore, the description is supplemented with the following examples:

**Example 1.** Preparation of tablets containing codeine and salt of magnesium (II)

Tablet cores with the following composition:

| | |
|---|---|
| - magnesium citrate x 9 $H_2O$: | 250 mg |
| - Codeine phosphate hemihydrate (COD) | 30 mg |
| - lactose monohydrate | 48.17 mg |
| - corn starch | 14.40 mg |
| - modified starch | 9.60 mg |
| - polyvinylpyrrolidone 25.000 | 4.00 mg |
| - magnesium stearate | 0.80 mg |

have been prepared by producing a granulate via wet granulation. Next, the remaining ancillary substances were added. Such a mixture produced was used to compress tablets on a rotary tabletter. Next, the tablets were coated with a polymer film. The resulting tablets demonstrate excellent mechanical parameters.

**Example 2.** Preparation of sachets with soluble granulate containing codeine and magnesium (II)

First, granulate containing components 1-4 has been prepared by producing a granulate via wet granulation. Next, the remaining ancillary substances were added. Such a mixture produced was used as sachet filling. The resulting granulate demonstrate excellent solubility in drinking watter (150-250 ml). It can also administrated orally in solid form, without prior dissolution in water.

A. Sachet containig 15/150 mg dose:

| Composition for 1 sachet | Dose [mg] | Amount of substance [mg] | |
|---|---|---|---|
| Codeine phosphate hemihydrate | 15 | 15.40* | 0.52% |
| Mg$^{2+}$jons 150 mg: Micronized magnesium citrate 9H2O | 150 | 1268.8* | 43.23% |
| VA64 | | 21.79 | 0.74% |
| Croscarmellose sodium | | 9 | 0.31% |
| Granulate summary | | 1315,0 | 44,80% |
| Mannitol,(Perlitol M 200) | | 1500 | 51,11% |
| Sucralose | | 10,00 | 0,34% |
| Cappuccino aroma | | 100 | 3,41% |
| Vanilla aroma | | 10 | 0,34% |
| | Sum: | 2935,00 | 100,00% |

B. Sachet containig 30/300 mg dose:

| Composition for 1 sachet | Dose [mg] | Amount of substance [mg] | |
|---|---|---|---|
| Codeine phosphate hemihydrate | 30 | 30.8* | 0,52% |
| Mg$^{2+}$jons 150 mg: Micronized magnesium citrate 9H2O | 300 | 2537,6* | 43,23% |
| VA64 | | 43,58 | 0,74% |

(continued)

| Composition for 1 sachet | Dose [mg] | Amount of substance [mg] | |
|---|---|---|---|
| Croscarmellose sodium | | 18 | 0,31% |
| Granulate summary | | 2630,0 | 44,80% |
| Mannitol,(Perlitol M 200) | | 3000 | 51,11% |
| Sucralose | | 20,00 | 0,34% |
| Cappuccino aroma | | 200 | 3,41% |
| Vanilla aroma | | 20 | 0,34% |
| | Sum: | 5870,00 | 100% |

**Example 3.** Assessment of antiinflammatory properties of combined codeine and magnesium in the rat model of carrageenan-induced paw oedema.

The aim of the presented studies was to assess anti-inflammatory properties of codeine (COD) combined with magnesium in the rat inflammatory model induced by carrageenan (CARR). CARR is a sulfated polysaccharide extracted from *Chondrus crispus,* a marine algae. It has been used for decades in research for its potential to induce inflammation in different animal models. CARR induces inflammation which promotes reactive oxygen species generation and neutrophil extracellular trap formation. These phenomena underlie oedema formation and lowering the nociceptive threshold [Barth et al., 2016]. To assess the effect of COD and magnesium on oedema formation, in rats, we used the plethysmometer method.

1. Materials and methods

1.1. *Animals and housing conditions*

The procedures for *in vivo* tests were approved by the 1st Local Ethics Committee of the Jagiellonian University in Krakow and the treatment of animals was in full accordance with ethical standards laid down in respective Polish and EU regulations (Directive No. 86/609/EEC). Experiments were carried out at the Department of Pharmacodynamics, Faculty of Pharmacy, Jagiellonian University Medical College in Krakow. The investigators involved in behavioral assays were blinded to experimental groups to avoid potential bias in data recording.

Adult male Krf: Wistar rats weighing 180-220 g were purchased from the Animal Breeding Farm of the Jagiellonian University at the Faculty of Pharmacy. Before behavioral tests the animals were kept in groups of 5 rats in standard plastic cages and housed under controlled conditions (room temperature of $22\pm2°$ C, light/dark (12:12) cycle, lights on at 8.00 AM, humidity $55\pm10\%$ and free access to food and water). Experimental groups consisted of 6-9 rats/dose. All experiments were performed between 9 AM and 3 PM. For the tests the animals were selected randomly. After the *in vivo* assay the animals were immediately euthanized.

1.2. *Chemicals and drug administration protocols*

Codeine phosphate hemihydrate (COD) and magnesium citrate x 9 $H_2O$ were purchased from MacFarlan Smith Ltd. (UK) and Dr. Paul Lohmann GmbH & Co. KGaA (Germany), respectively. COD and magnesium at various doses were orally administered to rats before behavioral tests. Doses of COD and magnesium were selected experimentally. CARR was supplied by Sigma Aldrich (Poznan, Poland).

In this study COD and magnesium were used alone or as combined treatment and they were administered as a single-dose treatment.

1.3. *Behavioral* tests

1.3.1. *Plethysmometer method*

In rats the antioedematous activity was assessed using the plethysmometer method. *CARR-induced paw oedema.* Before the assay rats were food-deprived overnight. The development of the paw oedema was measured plethysmographically (Ugo Basile Plethysmometer, Italy, Type 7140). The inflammation was induced according to the method described by Winter et al. (1962) and Lence (1962). Briefly, the rats were divided into groups, one of them being the control. In order to induce inflammation, 0.1 ml of 1% CARR sodium dissolved in 0.9% saline (Polfa Kutno, Poland) was injected into the plantar side of the right hind paw of each rat, 1 h after oral administration of COD. Physiological saline was administered to the control rats in the same way as the tested compounds. Paw volume was measured plethysmographically before the intraplantar CARR injection (zero time) and 3 h after CARR injection. Antioedematous activity was expressed as oedema increase 3 h after CARR (vs. baseline - before CARR administration).

*1.3.2. Data analysis*

Data analysis was carried out using GraphPad Prism software (v. 9.0, CA, USA). Numerical results are expressed as the mean $\pm$ SEM. Statistical analysis was carried out by using repeated measures ANOVA followed by Tukey's *post hoc* comparisons. $P < 0.05$ was considered significant.

Antioedematous activity was expressed as oedema increase 3 h after CARR (vs. baseline, i.e., before CARR administration).

2. Results

*2.1.1. Effect of test drugs on the oedema formation in rats (plethysmometer method)*

*2.1.1.1. Dose protocol - test compounds used alone*

In this assay repeated measures ANOVA revealed an overall significant effect of treatment ($F[19,307]=35.34$, $p < 0.0001$). Time effect and the drug x time interaction were also significant ($F[2,307]=410.7$, $p < 0.0001$ and $F[38,307]=4.187$, $p < 0.0001$, respectively). As shown in Fig. 1, 1% CARR induced paw oedema in rats. The paw oedema was increasing gradually in the course of the experiment.

In this assay, 3 h after CARR injection COD 120 and COD 240 mg/kg significantly ($p < 0.001$ and $p < 0.05$ vs. control, respectively) reduced CARR-induced paw oedema formation. Magnesium used alone at doses 30, 75 and 150 mg/kg did not show antioedematous properties in this test (Fig. 1).

*2.1.1.2. Dose protocol - test compounds used in combination*

As shown in Figs. 2-4, 1% CARR induced paw oedema in rats. The oedema was increasing gradually in the course of the experiment.

As shown in Figs. 2-4, this part of the study revealed that 3 h after CARR administration neither COD 15 mg/kg, nor magnesium 30, 75, 150 mg/kg, each used alone, were effective as antioedematous agents. In contrast to this, combined COD 15 mg/kg and magnesium at doses 30, 75 and 150 mg/kg significantly reduced paw oedema formation 3 h after CARR injection (COD 15 mg/kg + magnesium 30, 75 mg/kg: $p < 0.01$ and COD 15 mg/kg + magnesium 150 mg/kg: $p < 0.0001$ vs. control). Importantly, 3 h after CARR, COD 15 mg/kg combined with magnesium 150 mg/kg were significantly ($p < 0.05$) more effective as antioedematous agents than COD 15 mg/kg used alone (Fig. 4).

As shown in Figs. 5-7, COD 60 mg/kg used alone was not effective as an antioedematous agent 3 h after CARR administration. Compared to the control group, combined COD 60 mg/kg + magnesium 30 mg/kg was significantly ($p < 0.0001$) effective 3 h after CARR administration (Fig. 5). Combined COD 60 mg/kg + magnesium 75 mg/kg was significantly more effective than vehicle 3 h after CARR ($p < 0.01$, Fig. 6) and combined COD 60 mg/kg + magnesium 150 mg/kg was significantly more effective than vehicle 3 h after CARR injection ($p < 0.0001$, Fig. 7).

As shown in Fig. 5, combined COD 60 + magnesium 30 mg/kg did not reduce paw oedema more effectively than COD 60 mg/kg used alone. Also combined COD 60 + magnesium 75 mg/kg (Fig. 6) was not more effective in reducing paw oedema than COD 60 mg/kg used alone, either. In contrast to this, 3 h after CARR, combined COD 60 + magnesium 150 mg/kg reduced paw oedema more effectively than COD 60 mg/kg used alone ($p < 0.001$, Fig. 7).

As shown in Figs. 8-10, COD 120 mg/kg used alone significantly ($p < 0.001$) reduced paw oedema 3 h after CARR injection. Compared to the control group, combined COD 120 mg/kg + magnesium 30 mg/kg was significantly ($p < 0.0001$) effective 3 h after CARR administration. Combined COD 120 mg/kg + magnesium 75 mg/kg was significantly more effective ($p < 0.0001$) than vehicle 3 h after CARR injection. Also combined COD 120 mg/kg + magnesium 150 mg/kg was significantly ($p < 0.0001$) more effective than vehicle 3 h after CARR.

As shown in Fig. 8, 3 h after CARR, combined COD 120 mg/kg + magnesium 30 was more effective as an antioedematous agent than codeine 120 mg/kg used alone ($p < 0.05$). Also codeine 120 mg/kg combined with magnesium 75 or 150 mg/kg reduced paw oedema more effectively than COD 120 mg/kg used alone (significant at $p < 0.0001$, Figs. 9 and 10).

As shown in Figs. 11-13, COD 240 mg/kg used alone significantly ($p < 0.05$) reduced paw oedema 3 h after CARR injection. Compared to the control group, combined COD 240 mg/kg + magnesium 30, 75 and 150 mg/kg were significantly (p < 0.0001) more effective 3 h after CARR administration.

As shown in Figs. 11-13, 3 h after CARR, combined COD 240 mg/kg + magnesium 30, 75 and 150 mg/kg reduced paw oedema more effectively than COD 240 mg/kg used alone (significant at p < 0.0001).

**Table 1.** Antioedematous properties of COD, magnesium, each used alone or in combination, assessed in the rat model of inflammation induced by intraplantar CARR measured as effects on oedema reduction 3 h after CARR

| Treatment [mg/kg] | Oedema reduction (vs. control) |
|---|---|
| COD 15 | - |
| COD 60 | - |
| COD 120 | + |
| COD 240 | + |
| Mg 30 | - |
| Mg 75 | - |
| Mg 150 | - |
| COD 15 + Mg 30 | + |
| COD 15 + Mg 75 | + |
| COD 15 + Mg 150 | + |
| COD 60 + Mg 30 | + |
| COD 60 + Mg 75 | + |
| COD 60 + Mg 150 | + |
| COD 120 + Mg 30 | + |
| COD 120 + Mg 75 | + |
| COD 120 + Mg 150 | + |
| COD 240 + Mg 30 | + |
| COD 240 + Mg 75 | + |
| COD 240 + Mg 150 | + |
| **+:** active; -: not active | |

**Table 2.** Comparison of antioedematous properties of COD used alone or in combination with magnesium, assessed in the rat model of inflammation measured as effects on oedema reduction 3 h after CARR

| Parameter measured | Cod 15 + Mg 30 | Cod 15 + Mg 75 | Cod 15 + Mg 150 | Cod 60 + Mg 30 | Cod 60 + Mg 75 | Cod 60 + Mg 150 | Cod 120 + Mg 30 | Cod 120 + Mg 75 | Cod 120 + Mg 150 | Cod 240 + Mg 30 | Cod 240 + Mg 75 | Cod 240 + Mg 150 |
|---|---|---|---|---|---|---|---|---|---|---|---|---|
| | Vs. COD 15 | | | Vs. COD 60 | | | Vs. COD 120 | | | Vs. COD 240 | | |
| Oedema reduction | - | - | + | - | - | + | + | + | + | + | + | + |
| +: active; -: not active | | | | | | | | | | | | |

**Conclusions:**

- Codeine administered in higher doses of 120 and 240 mg/kg significantly decreased oedema after administration of CARR.

- Magnesium administered in a dose range of 30-150 mg/kg did not affect the CARR paw edema.

Although codeine at a doses of 15 or 60 mg/kg as well as magnesium at a dose of 150 mg/kg did not change the paw volume, the combination of codeine 15mg/kg + magnesium 150 mg/kg or codeine 60 mg/kg + magnesium 150 mg/kg significantly reduced the swelling of the paw.

- Magnesium administered at doses of 30, 75 or 150 mg/kg markedly intensified the anti-swelling effect of codeine at doses of 120 or 240 mg/kg.Conclusion:
  Magnesium markedly intensified the anti-inflammatory activity of codeine.

**Example 4. Assessment antinociceptive properties of combined codeine and magnesium in the model of carragenan- induced paw oedema**

In next experiments, the aim of the example 4, 5 and 6 was to investigate antinociceptive properties of codeine combined with magnesium in the inflammatory pain model induced by carrageenan (CARR), acetic acid (visceral pain) as well as in diabetic-neuropathy. To assess the effect of codeine and magnesium on mechanical nociceptive thresholds (CARR model, diabetic-neuropathy) the Randall-Selitto and von Frey test were used, respectively. To assess activity of investigated compounds in an inflammatory visceral pain induced by acetic acid the writhing test was used.

It is well known that one of the basic mechanisms responsible for the development of pain after administration of carrageenan, acetic acid and diabetic nerve damage is inflammation [Baum et al., 2021, Barth et al., 2016, Dai et al., 2021].

**1. Materials and methods**

**a.** *Animals and housing conditions*

The procedures for *in vivo* tests were approved by the 1st Local Ethics Committee of the Jagiellonian University in Krakow and the treatment of animals was in full accordance with ethical standards laid down in respective Polish and EU regulations (Directive No. 86/609/EEC). Experiments were carried out at the Department of Pharmacodynamics, Faculty of Pharmacy, Jagiellonian University Medical College in Krakow. The investigators involved in behavioural assays were blinded to experimental groups to avoid potential bias in data recording.

Adult male Krf: Wistar rats weighing 180-220 g were purchased from the Animal Breeding Farm of the Jagiellonian University at the Faculty of Pharmacy. Before behavioural tests the animals were kept in groups of 5 rats in standard plastic cages and housed under controlled conditions (room temperature of $22\pm2°$ C, light/dark (12:12) cycle, lights on at 8.00 AM, humidity $55\pm10\%$ and free access to food and water). Experimental groups consisted of 6-9 rats/dose. All experiments were performed between 9 AM and 3 PM. For the tests the animals were selected randomly. After the *in vivo* assay the animals were immediately euthanized.

b. *Chemicals and drug administration protocols*

Codeine phosphate hemihydrate and magnesium citrate x 9 $H_2O$ were purchased from MacFarlan Smith Ltd. (UK) and Dr. Paul Lohmann GmbH & Co. KGaA (Germany), respectively. Codeine and magnesium at various doses were orally administered to rats before behavioral tests. Doses of codeine and magnesium were selected experimentally. CARR was supplied by Sigma Aldrich (Poznan, Poland).

In this present study codeine and magnesium were used alone or as combined treatment and they were administered as a single-dose treatment.

c. *Behavioral* tests

i. *Randall - Selitto test*

Mechanical nociceptive threshold was assessed with the use of an analgesiameter (Ugo Basile Analgesy Meter, Italy, Type 37215) according to the method described by Randall and Selitto (1957). The mechanical nociceptive threshold of rats was assessed before drug administration (referred to as '0 min'; predrug) and then 3 h after CARR administration. At these time points, pressure was applied to the plantar surface of the rat's paw through a tip of an analgesiameter, to the point where the animal struggled, squealed or attempted to bite. This reaction (nociceptive flexion reflex) of the rat was quantified using the Randall-Selitto paw pressure device, which applied a linearly increasing mechanical force to the dorsum of the rat's hind paw. The mechanical nociceptive threshold was defined as the force (expressed in grams) at which the rat withdrew its paw and then the stimulus was stopped immediately. The cut off pressure was set to 260 g.

*ii. Data analysis*

Data analysis was carried out using GraphPad Prism software (v. 9.0, CA, USA). Numerical results are expressed as the mean ± SEM. Statistical analysis was carried out by using repeated measures ANOVA followed by Tukey's *post hoc* comparisons. P < 0.05 was considered significant.

In the Randall-Selitto test changes in pain threshold were calculated as % of baseline value (0 min - predrug pain threshold) according to the following formula:

$$\% \text{ of analgesia} = \text{postdrug/predrug} \times 100 - 100$$

where predrug is the baseline (at 0 min) pressure (expressed in [g]) that evokes animal's reaction, and postdrug is pressure (expressed in [g]) that evokes animal's reaction measured 3 h after CARR administration.

Percentage of analgesia values obtained as above for the individual animals were then used to calculate average (mean) values ± SEM in experimental groups.

## 2. Results

In the Randall-Selitto test, codeine 60 mg/kg used alone endured hyperalgesia 3h after CARR administration. Codeine 60 mg/kg combined with magnesium at a dose of 150 mg/kg showed statistically significant (p < 0.0001 vs. control) analgesic properties. Moreover, combined codeine at a dose of 60 mg/kg and magnesium at a dose of 150 mg/kg was significantly (p < 0.0001) more effective than codeine 60 mg/kg used alone (Fig 14).

**Exemple 5. Assessment antinociceptive properties of combined codeine and magnesium in the diabetic neuropathic pain model**

The aim of this study was to investigate antinociceptive properties of codeine phosphate combined with magnesium in the diabetic-neuropathy. To assess the effect of codeine and magnesium citrate on mechanical nociceptive thresholds von Frey test was used.

It is well known that one of the basic mechanisms responsible for the development of diabetic nerve damage and pain is inflammation [Baum et al., 2021].

## 1. Materials and methods

*1.1. Animals and housing conditions*

The *in vivo* study was carried out in mice. Adult male Albino Swiss (CD-1) mice were purchased from the Animal Breeding Farm of the Faculty of Pharmacy, Jagiellonian University Medical College in Krakow, Poland. At the beginning of the experiment the animals weighed between 18-22 g. The animals were housed in groups of 10 mice per cage at an ambient temperature of 22±2 °C, under a light/dark (12:12) cycle. Before the *in vivo* assays the animals had free access to food and water. The ambient temperature of the experimental room and humidity (55±10%) were kept constant throughout the tests. For the behavioral test the animals were selected randomly. Each experimental group consisted of 7-10 animals. Behavioral assays were performed between 8 AM and 2 PM. Immediately after the *in vivo* tests the animals were euthanized. All procedures for animal maintenance and treatment were approved by the 1st Local Ethics Committee of the Jagiellonian University in Krakow (Approval No. 487/2021) and the treatment of animals was in full accordance with ethical standards laid down in respective Polish and EU regulations (Directive No. 86/609/EEC).

*1.2. Chemicals and drug administration protocol*

Codeine phosphate hemihydrate and magnesium citrate x 9 $H_2O$ were purchased from MacFarlan Smith Ltd. (UK) and Dr. Paul Lohmann GmbH & Co. KGaA (Germany), respectively.

In the present study codeine and magnesium were used alone or as combined treatment and both agents were administered as a single-dose treatment.

*1.3. Behavioral tests*

*1.3.1. Induction of diabetes and selection of mice for further pain tests*

To induce type I diabetes, the mice were intraperitoneally injected with streptozotocin (STZ,a single injection, 200 mg/kg) dissolved in 0.1 N citrate buffer (Polskie Odczynniki Chemiczne, Poland). Age-matched control mice received an equal volume of citrate buffer (Tanabe et al., 2008).

Blood glucose level was measured 20 days after STZ injection. For this purpose a blood glucose monitoring system (AccuChek Active, Roche, France) was used. Blood samples were obtained from the tail vein of the mice. The animals were considered as diabetic when their blood glucose concentration exceeded 300 mg/dl (Tanabe et al., 2008) and only these mice (i.e., diabetic mice) were used in subsequent pain tests which were first performed 21 days after STZ administration.

*1.3.2. Effect on mechanical nociceptive threshold - von Frey test*

To assess the effect of codeine and magnesium on tactile allodynia in neuropathic (STZ-treated) animals von Frey test was used. Tactile allodynia was assessed using the electronic von Frey unit (Bioseb, France). This apparatus is supplied with a single flexible filament applying increasing force (from 0 to 10 g) against the plantar surface of the hind paw of a mouse. The paw withdrawal response automatically turns off the stimulus and the mechanical pressure that evoked the response is recorded.

On the day of the experiment, the mice were placed individually in test compartments with a wire mesh bottom and were allowed to habituate for 1 h. After the habituation period, in order to obtain baseline (predrug) values of pain sensitivity, each mouse was tested 3 times alternately in each hind paw, allowing at least 30 s between each measurement. Then, the mice were orally pretreated with codeine, magnesium or vehicle (via gastric tube) and then the animals were tested again to obtain postdrug values for each mouse (Salat et al., 2013).

*1.4. Data analysis*

Data analysis of the results was carried out using GraphPad Prism software (v. 9.0, CA, USA). For the statistical analysis one-way analysis of variance (ANOVA), followed by Tukey's *post-hoc* comparison, or repeated measures ANOVA, followed by Tukey's comparison were used. $P < 0.05$ was considered significant.

In the von Frey test changes in pain threshold were calculated as % of baseline value (at 0 min - predrug pain threshold) according to the following formula:

$$\% \text{ of analgesia} = (\text{postdrug}/\text{predrug}) \times 100 - 100$$

where predrug is the baseline (at 0 min) pressure (expressed in [gf]) that evokes animal's reaction, and postdrug is pressure (expressed in [gf]) that evokes animal's reaction measured after drug administration.

Percentage of antinociception values obtained as above for the individual animals were then used to calculate average (mean) values $\pm$ SEM in experimental groups.

## 2. Results

Codeine at a dose of 60 mg/kg used alone reduced tactile allodynia in diabetic, STZ-treated mice. Codeine at a dose of 60 mg/kg combined with magnesium at doses of 30, 75 and 150 mg/kg not only significantly reversed tactile allodynia but clear antinociceptive effect occured (significant at $p < 0.0001$ vs. diabetic control). Importantly, all three combinations of codeine at a dose of 60 mg/kg with magnesium (30, 75 and 150 mg/kg) were significantly more effective than codeine 60 mg/kg used alone ($p < 0.05$; Fig. 15).

## Example 6. Assessment antinociceptive properties of combined codeine and magnesium in the inflammatory visceral pain model.

The aim of this study was to investigate antinociceptive properties of codeine combined with magnesium in the inflammatory pain model induced by acetic acid. To assess activity of investigated compounds in an inflammatory visceral pain the writhing test was used.

It is well known that one of the basic mechanisms responsible for the development of pain after intraperitoneal administration of acetic acid is inflammation [Dai et al., 2021].

## 1. Materials and methods

### a. *Animals and housing conditions*

The procedures for *in vivo* tests were approved by the 1st Local Ethics Committee of the Jagiellonian University in Krakow and the treatment of animals was in full accordance with ethical standards laid down in respective Polish and EU regulations (Directive No. 86/609/EEC). Experiments were carried out at the Department of Pharmacodynamics, Faculty of Pharmacy, Jagiellonian University Medical College in Krakow. The investigators involved in behavioural assays were blinded to experimental groups to avoid potential bias in data recording.

Adult male Albino Swiss CD-1 mice weighing 18-22 g were purchased from the Animal Breeding Farm of the Jagiellonian University at the Faculty of Pharmacy. Before behavioural tests the animals were kept in groups of 10 mice in standard plastic cages and housed under controlled conditions (room temperature of $22\pm2°$ C, light/dark (12:12) cycle, lights on at 8.00 AM, humidity $55\pm10\%$ and free access to food and water). Experimental groups consisted of 6 - 10 mice/dose. All experiments were performed between 9 AM and 3 PM. For the tests the animals were selected randomly. After the *in vivo* assay the animals were immediately euthanized.

### b. *Chemicals and drug administration protocols*

Codeine phosphate hemihydrate and magnesium citrate x 9 $H_2O$ were purchased from MacFarlan Smith Ltd. (UK) and Dr. Paul Lohmann GmbH & Co. KGaA (Germany), respectively. Both agents at various, experimentally selected doses, each drug used alone or in combination, were administered orally. Acetic acid was supplied by Polskie Odczynniki Chemiczne (Gliwice, Poland).

### c. *Behavioral test - writhing test*

In the writhing test the mice were placed individually into glass beakers and were allowed to habituate for the next 30 min. Then, each mouse was weighed, injected with the test compound or vehicle and then placed back into the glass beaker until the administration of acetic acid solution. To induce inflammatory pain, 0.9% acetic acid solution prepared in distilled water was injected by the intraperitoneal route. After that, the mice were placed back in the beakers and were observed continuously for the next 30 min. Stereotypical writhes (lengthwise constrictions of the torso with a concomitant concave arching of the back) were counted over this period in drug-treated and control mice [Salat et al., 2018].

### i. *Data analysis*

Data analysis was carried out using GraphPad Prism software (v. 9.0, CA, USA). Numerical results are expressed as the mean $\pm$ SEM. Statistical analysis was carried out by using one-way ANOVA followed by Tukey's *post hoc* comparison. $P < 0.05$ was considered significant.

## 3. Results

Although codeine at a dose of 30 mg/kg used alone was not significantly effective in the mouse writhing test, the addition of magnesium at doses of 30 mg/kg or 75 mg/kg to codeine 30 mg/kg resulted in a statistically significant antinociceptive effect ($p < 0.0001$ vs. vehicle control). Importantly, both drug combinations were also significantly more effective than codeine at a dose of 30 mg/kg used alone ($p < 0.05$ and $p < 0.01$, respectively) (Fig. 16).

## References

Barth CR, Funchal GA, Luft C, de Oliveira JR, Porto BN, Donadio MV. Carrageenan-induced inflammation promotes ROS generation and neutrophil extracellular trap formation in a mouse model of peritonitis. Eur J Immunol. 2016 Apr;46(4):964-70. doi: 10.1002/eji.201545520

Baum P, Toyka KV, Blüher M, Kosacka J, Nowicki M. Inflammatory Mechanisms in the Pathophysiology of Diabetic Peripheral Neuropathy (DN)-New Aspects. Int J Mol Sci. 2021 Oct 7;22(19):10835. doi: 10.3390/ijms221910835

Dai G, Li B, Xu Y, Li Z, Mo F, Wei C. Synergistic interaction between matrine and paracetamol in the acetic acid writhing test in mice. Eur J Pharmacol. 2021 Mar 15;895:173869. doi: 10.1016/j.ejphar.2021.173869.

Lence P (1962). A new device for plethysmoscopic measuring of small objects. Arch Int Pharmacodyn Ther 136:237-40.

Libako P, Nowacki W, Castiglioni S, Mazur A, Maier JA. Extracellular magnesium and calcium blockers modulate macrophage activity. Magnes Res. 2016 Mar 1;29(1):11-21. doi: 10.1684/mrh.2016.0398.

Liu CC, Lu IC, Wang LK, Chen JY, Li YY, Yang CP, Liu PH, Cheng WJ, Tan PH. Interferon-β suppresses inflammatory pain through activating μ-opioid receptor. Mol Pain. 2021 Jan-Dec;17:17448069211045211. doi: 10.1177/17448069211045211. Medzhitov R. Inflammation 2010: new adventures of an old flame. Cell. 2010 Mar 19;140(6):771-6. doi: 10.1016/j.cell.2010.03.006.

Mogilski S, Kubacka M, Lazewska D, Wiecek M, Gluch-Lutwin M, Tyszka-Czochara M, Bukowska-Strakova K, Filipek B, Kiec-Kononowicz K. Aryl-1,3,5-triazine ligands of histamine H4 receptor attenuate inflammatory and nociceptive response to carrageen, zymosan and lipopolysaccharide. Inflamm Res. 2017; 66(1):79-95. doi: 10.1007/s00011-016-0997-z.

Randall LO, Selitto JJ. A method for measurement of analgesic activity of inflamed tissue. Arch Int Pharmacodyn. 1957; 111:409-19.

Ronchetti S, Migliorati G, Delfino DV. Association of inflammatory mediators with pain perception. Biomed Pharmacother. 2017 Dec;96:1445-1452. doi: 10.1016/j.biopha.2017.12.001..

Simon LS, Taylor PC, Choy EH, Sebba A, Quebe A, Knopp KL, Porreca F. The Jak/STAT pathway: A focus on pain in rheumatoid arthritis. Semin Arthritis Rheum. 2021 Feb;51(1):278-284. doi: 10.1016/j.semarthrit.2020.10.008.

Stein C. Targeting pain and inflammation by peripherally acting opioids. Front Pharmacol. 2013 Sep 23;4:123. doi: 10.3389/fphar.2013.00123.

Winter CA, Risley EA, Nuss GW (1962). Carrageenin-induced edema in the hind paw of the rat as an assay for anti-inflammatory drugs. Proc Soc Exp Biol Med 111:544-7. Zhang X, Chen Q, Mao X. Magnesium Enhances Osteogenesis of BMSCs by Tuning Osteoimmunomodulation. Biomed Res Int. 2019 Nov 14;2019:7908205. doi: 10.1155/2019/7908205

**Claims**

1. A pharmaceutical composition for use in treatment of inflammation, comprising an opioid and a pharmaceutically admissible compound of magnesium (II), wherein presence of said magnesium (II) increases the anti-inflammatory activity of said opioid.

2. A composition for use according to claim 1, **characterised in that** it is for oral treatment.

3. A composition for use according to claim 1, **characterised in that** the compound of magnesium (II) is salt, possibly selected from a group consisting of: magnesium chloride, magnesium lactate, magnesium hydroaspartate, magnesium citrate or hydrates thereof.

4. A composition for use according to claim 1, **characterised in that** the salt of magnesium (II) is magnesium citrate or hydrate thereof, possibly magnesium citrate x 9 $H_2O$.

5. A composition for use according to claim 1, **characterised in that** the opioid is selected from a group consisting of: dihydrocodeine, oxycodone or phenantrene derivatives of opium such as: morphine, codeine, or another opioid such as tramadol.

6. A composition for use according to claim 1, **characterised in that** the opioid is in the form of pharmaceutically admissible salt thereof.

7. A composition for use according to claim 1, **characterised in that** the opioid is codeine, their salt or hydrate thereof, possibly codeine phosphate hemihydrate.

8. A composition for use according to claim 1, **characterised in that** the opioid is used in dose of 15 mg/kg to 240 mg/kg.

9. A composition for use according to claim 1, **characterised in that** the opioid is used in dosage unit containing 15mg or 30 mg of codeine.

10. A composition for use according to claim 1, **characterised in that** the magnesium (II) is used in dose of 30 mg/kg to 300 mg/kg.

11. A composition for use according to claim 1, **characterised in that** the magnesium (II) is used in dosage unit containing 150mg or 300 mg of magnesium (II).

12. A composition for use according to claim 1, **characterised in that** the inflammation is resulting from any ailments with an inflammatory component, in particular such as neuropathic pain, inflammatory pain or visceral pain.

13. A composition for use according to claim 1, **characterised in that** the inflammation is manifested by oedema.

**Fig. 1**

Fig. 2

Fig. 3

Fig. 4

Fig. 5

Fig. 6

Fig. 7

Fig. 8

Fig. 9

Fig. 10

Fig. 11

Fig. 12

Fig. 13

**Fig. 14**

Fig. 15

Fig. 16

Europäisches Patentamt

European Patent Office

Office européen des brevets

# EUROPEAN SEARCH REPORT

Application Number

EP 22 46 1646

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2012/165985 A1 (UNIV WARSZAWSKI MEDYCZNY [PL]; BUJALSKA-ZADROZNY MAGDALENA [PL] ET AL.) 6 December 2012 (2012-12-06) * page 15, paragraph 6; claim 1; examples 1,2 * * page 2, paragraph 3 * | 1-13 | INV. A61K31/135 A61K31/485 A61K33/06 A61K45/06 A61P43/00 |
| Y | JP S64 16 A (SANTEN PHARMACEUTICAL CO LTD) 5 January 1989 (1989-01-05) * paragraph [0003]; figure 1 * | 1-13 | |
| Y | MAURO BIANCHI ET AL: "Effects of tramadol on experimental inflammation", FUNDAMENTAL & CLINICAL PHARMACOLOGY, ELSEVIER, PARIS, FR, vol. 13, no. 2, 26 August 2009 (2009-08-26), pages 220-225, XP071690594, ISSN: 0767-3981, DOI: 10.1111/J.1472-8206.1999.TB00342.X * Abstract and section 2.3; figure 1 * * page 222, column 2, lines 1-4 * | 1-13 | |
| Y | BEGON SOPHIE ET AL: "Magnesium increases morphine analgesic effect in different experimental models of pain", ANESTHESIOLOGY, AMERICAN SOCIETY ANESTHESIOLOGISTS, US, vol. 96, no. 3, 1 March 2002 (2002-03-01), pages 627-632, XP009161719, ISSN: 0003-3022, DOI: 10.1097/00000542-200203000-00019 * Abstract; page 628, column 1, paragraph 3 * * page 631, column 2, paragraph 1 * | 1-13 | TECHNICAL FIELDS SEARCHED (IPC) A61K A61P |

The present search report has been drawn up for all claims

| Place of search | Date of completion of the search | Examiner |
|---|---|---|
| Munich | 8 May 2023 | Allnutt, Sarah |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

..............................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 22 46 1646**

This annex lists the patent family members relating to the patent documents cited in the above-mentioned European search report.
The members are as contained in the European Patent Office EDP file on
The European Patent Office is in no way liable for these particulars which are merely given for the purpose of information.

**08-05-2023**

| Patent document cited in search report | | Publication date | Patent family member(s) | | Publication date |
|---|---|---|---|---|---|
| WO 2012165985 | A1 | 06-12-2012 | DK | 2714042 T3 | 02-01-2019 |
| | | | EP | 2714042 A1 | 09-04-2014 |
| | | | ES | 2708689 T3 | 10-04-2019 |
| | | | HU | E040419 T2 | 28-03-2019 |
| | | | PL | 2714042 T3 | 29-03-2019 |
| | | | US | 2014127302 A1 | 08-05-2014 |
| | | | WO | 2012165985 A1 | 06-12-2012 |
| JP S6416 | A | 05-01-1989 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BARTH CR ; FUNCHAL GA ; LUFT C ; DE OLIVEI-RA JR ; PORTO BN ; DONADIO MV.** Carrageen-an-induced inflammation promotes ROS generation and neutrophil extracellular trap formation in a mouse model of peritonitis. *Eur J Immunol.,* April 2016, vol. 46 (4), 964-70
- **BAUM P ; TOYKA KV ; BLÜHER M ; KOSACKA J ; NOWICKI M.** Inflammatory Mechanisms in the Pathophysiology of Diabetic Peripheral Neuropathy (DN)-New Aspects. *Int J Mol Sci.,* 07 October 2021, vol. 22 (19), 10835
- **DAI G ; LI B ; XU Y ; LI Z ; MO F ; WEI C.** Synergistic interaction between matrine and paracetamol in the acetic acid writhing test in mice. *Eur J Pharmacol.,* 15 March 2021, vol. 895, 173869
- **LENCE P.** A new device for plethysmoscopic measuring of small objects. *Arch Int Pharmacodyn Ther,* 1962, vol. 136, 237-40
- **LIBAKO P ; NOWACKI W ; CASTIGLIONI S ; MA-ZUR A ; MAIER JA.** Extracellular magnesium and calcium blockers modulate macrophage activity. *Magnes Res,* 01 March 2016, vol. 29 (1), 11-21
- **LIU CC ; LU IC ; WANG LK ; CHEN JY ; LI YY ; YANG CP ; LIU PH ; CHENG WJ ; TAN PH.** Interferon-β suppresses inflammatory pain through activating μ-opioid receptor. *Mol Pain,* January 2021, vol. 17
- **MEDZHITOV R.** Inflammation 2010: new adventures of an old flame. *Cell,* 19 March 2010, vol. 140 (6), 771-6

- **MOGILSKI S ; KUBACKA M ; LAZEWSKA D ; WIECEK M ; GLUCH-LUTWIN M ; TYSZKA-CZO-CHARA M ; BUKOWSKA-STRAKOVA K ; FILIPEK B ; KIEC-KONONOWICZ K.** Aryl-1,3,5-triazine ligands of histamine H4 receptor attenuate inflammatory and nociceptive response to carrageen, zymosan and lipopolysaccharide. *Inflamm Res,* 2017, vol. 66 (1), 79-95
- **RANDALL LO ; SELITTO JJ.** A method for measurement of analgesic activity of inflamed tissue. *Arch Int Pharmacodyn,* 1957, vol. 111, 409-19
- **RONCHETTI S ; MIGLIORATI G ; DELFINO DV.** Association of inflammatory mediators with pain perception. *Biomed Pharmacother,* December 2017, vol. 96, 1445-1452
- **SIMON LS ; TAYLOR PC ; CHOY EH ; SEBBA A ; QUEBE A ; KNOPP KL ; PORRECA F.** The Jak/STAT pathway: A focus on pain in rheumatoid arthritis. *Semin Arthritis Rheum,* February 2021, vol. 51 (1), 278-284
- **STEIN C.** Targeting pain and inflammation by peripherally acting opioids. *Front Pharmacol,* 23 September 2013, vol. 4, 123
- **WINTER CA ; RISLEY EA ; NUSS GW.** Carrageen-in-induced edema in the hind paw of the rat as an assay for anti-inflammatory drugs. *Proc Soc Exp Biol Med,* 1962, vol. 111, 544-7
- **ZHANG X ; CHEN Q ; MAO X.** Magnesium Enhances Osteogenesis of BMSCs by Tuning Osteoimmu-nomodulation. *Biomed Res Int.,* 14 November 2019, vol. 2019, 7908205